Europäisches Patentamt

European Patent Office

Office européen des brevets

(19)

(11) **EP 0 647 716 B1**

(12) **FASCICULE DE BREVET EUROPEEN**

(45) Date de publication et mention
de la délivrance du brevet:
**24.09.2003 Bulletin 2003/39**

(51) Int Cl.$^7$: **C12N 15/85**, C12N 15/11,
C12N 15/34, C12N 9/00,
C12N 15/86, C12N 5/10,
C07K 14/16, A61K 48/00

(21) Numéro de dépôt: **93401745.0**

(22) Date de dépôt: **06.07.1993**

(54) **Vecteur comportant un gène viral transcrit par l'ARN polymérase III**

Vektor, enthält ein bei ARN-Polymerase-III transcriptes Virus-Gen

Vector comprising viral gene transcribed by ARN polymerase III

(84) Etats contractants désignés:
**AT BE CH DE DK ES FR GB GR IE IT LI LU MC NL
PT SE**

(43) Date de publication de la demande:
**12.04.1995 Bulletin 1995/15**

(73) Titulaire: **UNIVERSITE DE NICE-SOPHIA
ANTIPOLIS
06100 Nice (FR)**

(72) Inventeurs:
• **Doglio, Alain
F-06730 Saint André (FR)**
• **Lefèbvre, Jean-Claude
F-06340 Drap (FR)**
• **Cagnon, Laurence
F-06100 Nice (FR)**

(74) Mandataire: **Warcoin, Jacques et al
Cabinet Régimbeau
20, rue de Chazelles
75847 Paris cedex 17 (FR)**

(56) Documents cités:
**EP-A- 0 387 775**          **FR-A- 2 687 411**

• **JOURNAL CELLULAR BIOCHEMISTRY,
vol.SUP0, no.17 E, 29 Mars 1993 page 200
DOGLIO, A. ET AL. 'Adenovirus VA-1 RNA as a
potential shuttle molecule to vehicle antisense
RNA against Human Immunodeficiency Virus'**
• **EMBO JOURNAL., vol.6, no.10, 1987, EYNSHAM,
OXFORD GB pages 3043 - 3047 JENNINGS, P.A.
ET MOLLOY, P.L. 'Inhibition of SV40 replicon
function by engineered antisense RNA
transcribed by polymerase III'**
• **EMBO JOURNAL., vol.8, no.12, 1989, EYNSHAM,
OXFORD GB pages 3861 - 3866 COTTEN, M. ET
BIRNSTIEL, M.L. 'Ribozyme mediated
destruction of RNA in vivo'**

EP 0 647 716 B1

**Description**

La présente invention concerne des vecteurs d'ADN recombinants, notamment de type plasmidique ou viral, comportant une cassette de transcription par l'ARN polymérase III constituée par un gène viral transcrit naturellement par l'ARN polymérase III.

La présente invention concerne aussi des cellules eucaryotes transfectées ou infectées par des vecteurs selon l'invention.

La présente invention concerne également un procédé de production intracellulaire d'un fragment d'ARN in vitro ou in vivo par culture de cellules eucaryotes transfectées ou infectées par les vecteurs selon l'invention.

La présente invention concerne également l'utilisation de vecteurs selon l'invention à titre de médicament.

Les molécules antisens sont des séquences d'ARN qui s'hybrident de manière sélective aux ARN messagers dont elles sont complémentaires et bloquent ainsi l'expression du gène considéré (maturation de l'ARN, traduction). Les travaux réalisés depuis le début des années 80 attestent de l'efficacité d'un tel système pour réprimer de nombreuses fonctions cellulaires ou virales (1,2).

Les perspectives d'applications potentielles de ces molécules antisens sont importantes en recherche fondamentale. L'utilisation des antisens pour corriger l'expression de certains phénotypes "anormaux" ou pour lutter efficacement contre des virus pour lesquels les approches classiques (vaccination, chimiothérapie...) s'avèrent délicates, comme cela est le cas pour le virus de l'Immunodéficience Humaine (HIV).

Un des problèmes posés par l'utilisation des antisens réside dans le fait que ces molécules sont efficaces à des concentrations intracellulaires importantes bien largement supérieures à celles de leur substrat. La concentration intracellulaire de molécules antisens étant le résultat de la différence entre la synthèse et la dégradation, on a recherché selon l'invention des solutions favorisant la synthèse et la stabilité des produits formés. Dans cette optique, l'efficacité de fonctionnement de la molécule antisens vis à vis de sa cible est un paramètre important puisque plus une molécule sera "puissante" moins elle nécessitera une concentration élevée pour obtenir un effet maximum.

L'ARN polymérase III est chez les eucaryotes l'enzyme responsable de la synthèse d'une grande variété de petits ARN cytoplasmiques ou nucléaires. Les principaux représentants étant les ARN de transfert (tRNA) ou les ARN ribosomiques de type 5S (3).

L'ARN polymérase III est capable de transcrire efficacement en système acellulaire des fragments d'ADN clonés. Ceci est possible à la condition que ces fragments d'ADN contiennent les régions promoteur spécifiques à la transcription par la polymérase III. Ces régions promoteur sont à présent bien caractérisées (4) et sont avant tout constituées par des régions intragéniques disposées de manière discontinue. Dans le cas des gènes de type tRNA, il s'agit de deux régions d'ADN : la "box A" et la "box B" chacune formée par 11 nucléotides (séquence consensus) et espacées par une région intermédiaire de taille variable (4).

Les ARN transcrits par la polymérase III sont remarquables par leur petite taille et surtout par leur conformation dans l'espace. Ainsi la structure en feuille de trèfle des tRNA est particulièrement bien connue. Cette structure secondaire assure vraisemblablement aux tRNA stabilité mais surtout fonctionnalité. Les séquences présentes au niveau des boucles (anticodon) sont libres de s'hybrider sur un ARN complémentaire. Cotten et Birnstiel ont en 1989 (5) proposé d'insérer une séquence "ribozyme/antisens" dans le gène tRNA[met] de Xenopus. L'oligonucléotide a été inséré dans la région intermédiaire située entre les box A et box B des régions promoteur de façon à être correctement présenté au niveau de l'anticodon (ribtRNA[met], référence 2).

Certains virus (Adénovirus, Epstein Barr virus, Herpes virus) sont dotés de gènes transcrits par l'ARN polymérase III. En particulier, les Adénovirus sont dotés de deux gènes, d'organisation similaire, appelés VAI et VAII (VA : Virus Associated) codant pour deux VA-ARN différents VA-ARNI et VA-ARNII (6). Les gènes VA sont naturellement clonés et fonctionnels dans le génome des Adénovirus. On trouve plus de $10^7$ molécules VA-ARN dans une cellule infectée par un Adénovirus. Ces petits ARN, comportant 150 nucléotides, sont transcrits à partir d'un promoteur comportant deux régions distinctes (box A et box B) localisées dans la région L1 du génome de l'Adénovirus (7). Les VA-ARN exercent un effet au niveau de la traduction : les mutants d'Adénovirus défectifs en VA-ARNI expriment normalement leurs ARN messagers mais sont incapables de les faire traduire efficacement. L'explication de ce phénomène est que la protéine kinase DAI induite par l'interféron est inhibée par la VA-ARNI, ce qui permet à son substrat eIF-2 d'échapper à la phosphorylation et donc à l'inactivation (9) (le facteur eIF-2 étant nécessaire à l'initiation de la traduction). L'interaction entre le VA-ARNI et la kinase a été bien documentée (10,11). Ils ont décrit deux régions distinctes de l'ARN, l'une étant impliquée dans la liaison de l'ARN avec la protéine DAI (nucléotides 93 à 136) et l'autre dans l'inhibition de l'activité kinasique (nucléotides 54 à 77).

Jennings et Molloy (12) ont montré qu'il était possible de réprimer l'expression d'un réplicon du virus SV40, dans les cellules COS1, grâce à un antisens anti SV40 de 163 pb (antigène T) greffé à l'extrémité 3' du gène VAI. Après transfection des cellules COS1 par diverses constructions (sens, antisens), les auteurs montrent sur un système d'expression transitoire que l'antigène T est réprimé à 50%.

On a, selon la présente invention, inséré une courte séquence antisens à l'intérieur du gène VA, dans le but d'obtenir un ensemble fonctionnel, c'est-à-dire conservant la structure partiellement double brin du gène VA et sa conformation à boucle, du type épingle à cheveux, qui interagit avec la protéine kinase. Ce maintien de la configuration structurelle se traduit par une plus grande stabilité intracellulaire, d'une part et une conservation de la fonctionnalité du gène VA, d'autre part.

On a en effet, selon la présente invention, découvert que l'efficacité remarquable du fonctionnement de ces petits ARN dans leur rôle d'inhibition de l'activité de la protéine kinase DAI peut être utilisée en le détournant de sa vocation première et en le dirigeant vers une autre cible par le biais des antisens ou des ribozymes. Selon l'invention, on utilise donc ces gènes VA comme système navette, en particulier pour le développement d'une nouvelle famille de molécules d'ARN antisens exprimées chez les eucaryotes et potentiellement utilisables pour une large gamme d'applications.

On a, selon la présente invention, développé un modèle de gène "cassettes" permettant l'insertion aisée d'oligonucléotides antisens ou de ribozymes dans le gène VA sans en affecter le taux de transcription et on a pu mesurer l'efficacité relative de ces constructions en matière d'inhibition de la réplication du virus HIV en culture. La présente invention repose en effet sur l'utilisation de l'ARN Polymérase III pour transcrire efficacement des gènes clonés (VAI) porteurs de séquences antisens ou ribozymes (sous forme de fragments d'ADN exogènes de petites tailles -15 à 25 nucléotides- insérés dans le gène considéré).

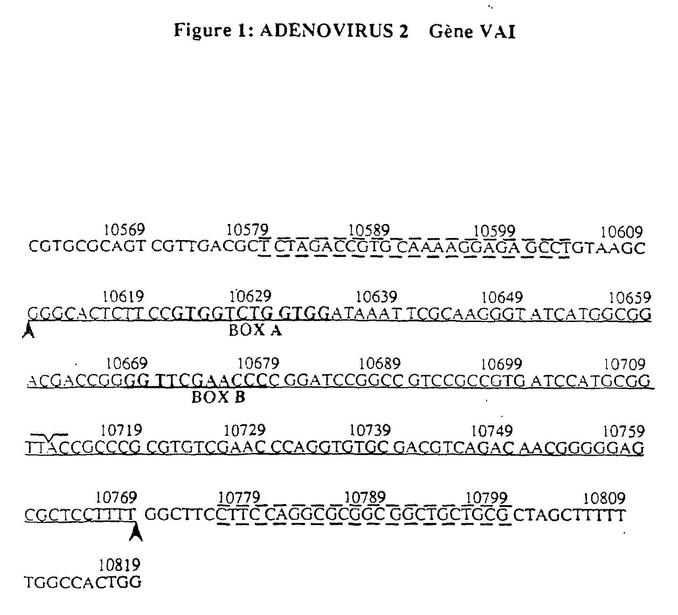

Dans son aspect le plus général, la présente invention a pour objet un vecteur d'ADN recombinant comportant une cassette de transcription par l'ARN polymérase III, constitué par le gène VAI d'adénovirus transcrit par l'ARN polymérase III dans lequel on a inséré un oligonucléotide, fragment d'ADN, dans la région allant des nucléotides 10694 à 10730 dudit gène VAI de l'Adénovirus 2 représenté Figure 1, ou à la place de la région 10694 à 10730, celle-ci étant délétée.

Le taux de transcription des gènes VA par la polymérase III est très important. Cet enzyme est bien conservé chez la plupart des eucaryotes et de ce fait adaptable à de nombreux modèles cellulaires ou animaux, la représentation ubiquitaire de cet enzyme dans tous les tissus ne pose pas de limitation tissulaire spécifique.

L'utilisation de ces gènes viraux transcrits en grande quantité, stables et fonctionnels, en fait donc des systèmes de production in situ d'ARN particulièrement efficaces.

L'organisation des gènes transcrits par cet enzyme est intéressante de par l'absence de séquences d'ADN régulatrices en position "extragénique", ce qui simplifie le schéma de régulation de la transcription et assure un compactage de l'information génétique. La position intragénique des promoteurs évite d'avoir à manipuler des régions non transcrites, ce qui permet de manipuler de petits gènes faciles à cloner. Enfin, les gènes viraux selon l'invention, tels que VA, offrent de nombreuses possibilités d'insertion d'oligonucléotides sans affecter leur taux de transcription et la structure secondaire de l'ARN transcrit.

Dans un mode de réalisation approprié, le gène viral est un gène VAI d'un adénovirus.

En particulier, on peut citer le gène VAI d'un Adénovirus, notamment l'Adénovirus 2.

Le gène VAI des Adénovirus a été choisi comme système de réalisation de l'invention, car il est particulièrement bien décrit dans la littérature. Cependant, l'ensemble des gènes viraux semblables au VAI transcrits par l'ARN Polymérase III peut également convenir.

Comme vecteur approprié selon l'invention, on peut citer un vecteur réplicatif plasmidique ou épisomique ou un vecteur viral.

En particulier, on utilisera un vecteur épisomique portant l'origine de réplication du virus Epstein Barr (oriP), ainsi que les séquences codantes pour la protéine EBNA-1.

S'agissant du choix du vecteur, il est en effet, préférable de rester le plus proche possible du système "naturel" en le perturbant le moins possible. Pour cette raison, on préfère utiliser un vecteur se répliquant de manière autonome (épisome). Le recours à un vecteur à réplication épisomique peut être un bon moyen pour exprimer des antisens dans des cellules eucaryotes et tout particulièrement dans le lymphocyte T. Le clonage des gènes VA/antisens dans des vecteurs se répliquant sous forme épisomique en système eucaryote est particulièrement approprié. Ces vecteurs portent l'origine de réplication du virus Epstein Barr (OriP) ainsi que des séquences codantes pour la protéine Ebnal strictement nécessaire à la réplication de la molécule d'ADN portant Ori P.

On note une plus grande efficacité de fonctionnement de la polymérase III sur des épisomes ainsi qu'une stimulation de l'activité transcriptionnelle en présence de la protéine Ela. Ces propriétés sont mises à profit pour améliorer le système proposé.

Le vecteur viral selon l'invention est de préférence un virus à ADN, mais peut également être un vecteur rétroviral à ARN ; il comportera alors le transcrit ARN de la cassette de transcription selon l'invention. Compte tenu du fait que l'infection d'une cellule par un rétrovirus entraîne la production d'un ADN proviral circularisé, c'est cet ADN proviral qui sera à son tour transcrit par l'ARN polymérase III conformément à l'invention, ceci n'excluant pas le fonctionnement de la construction génétique utilisée une fois que le DNA rétroviral est intégré.

Dans un mode de réalisation approprié selon l'invention, l'oligonucléotide est inséré dans la région allant des nucléotides 10694 à 10730 du gène VAI.

Pour une application thérapeutique, de préférence, le gène VA est inactivé en ce qui concerne l'inhibition de l'action de l'interféron, par délétion ou mutation dans le gène VA des éléments de séquence critiques pour l'inactivation de la protéine kinase, DAI, induite par l'interféron.

Cette inactivation peut se faire par insertion directe de l'oligonucléotide dans les régions responsables de l'activité de l'inhibition de l'action de l'interféron, région décrite pour être dans le gène VA I de l'Adénovirus 2 entre et y compris les nucléotides 10672 et 10745. Par ce biais également, on optimise l'affinité de l'oligonucléotide pour la séquence-cible, compte tenu de la configuration spatiale de cette région en forme de boucle.

L'intégrité de la région centrale du gène VA des nucléotides 10694 à 10730 (boucle IV) est en effet critique pour le maintien de l'activité inhibitrice de l'ARN VA vis à vis de la P68 kinase. Cette région est située en dehors des zones régulatrices (boîtes A et B) et sa délétion n'affecte pas la transcription du gène VA. Le document EP 387775 , qui mentionne l'integration d'oligonucléotides dans un gène, ne donne ancune précision sur les régions à choisir pour une intégration autre qu'entre les boites A et B du gène transcrit par l'ARN polymérase III.

Selon un premier mode de réalisation de la présente invention, on inactive l'activité naturelle du gène VA I de l'Adénovirus 2 par l'insertion de l'antisens dans la région centrale des nucléotides 10694 à 10730, plus particulièrement 10702 à 10728, ou à la place de cette région qui a été délétée.

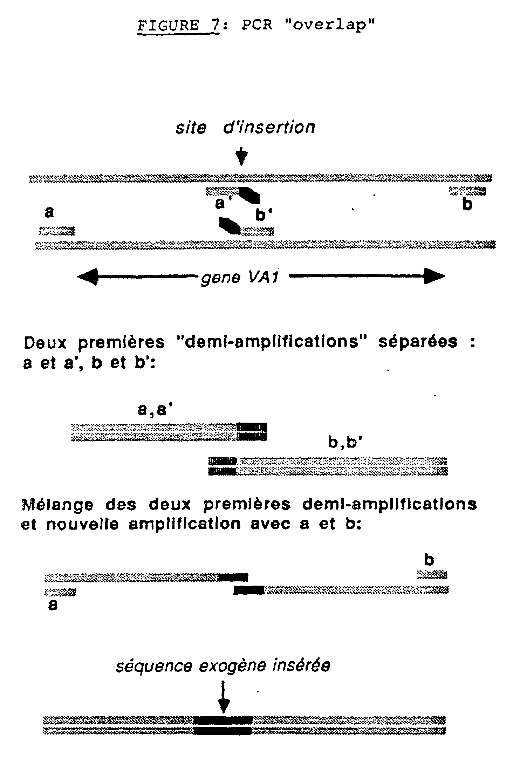

Cependant, afin de pouvoir construire des gènes chimères VA-antisens pour lesquels l'antisens est introduit en dehors de cette région, dans un autre mode de réalisation, on construit un gène VA délété de la région centrale (nucléotides 10702 à 10728). Pratiquement, La PCR "overlap" (figure 7) permet de réaliser ces délétions quand les oligonucléotides a' et b' sont espacés de la région a déléter. De plus, un site de restriction nouveau (EcoRV ; GATATC) peut être crée notamment par la juxtaposition des deux extrémités encadrant la délétion (GAT et ACC) grâce à la mutation du nucléotide 10729 (ACC remplacé par ATC). Ce site supplémentaire peut servir ultérieurement au clonage de séquences antisens ou ribozymiques en lieu et place de la boucle IV délétée. Ce gène est appelé VA delta IV.

Dans un mode de réalisation particulier, l'oligonucléotide est inséré au niveau du nucléotide 10711 du gène VAI de l'Adénovirus 2 représenté Figure 1.

De préférence, l'oligonucléotide selon l'invention comporte de 15 à 40 nucléotides, de préférence encore 15 à 25.

Dans le cadre d'une application thérapeutique, l'oligonucléotide pourra correspondre à une molécule ARN anti-sens ou à une structure d'ARN ribozymique.

Dans le but d'améliorer l'efficacité d'hybridation entre la molécule ARN antisens et son substrat, de nombreux travaux sont actuellement orientés vers la recherche de séquences cibles correctement définies en termes de paramètres d'hybridation (affinité, accessibilité).

Les séquences ribozymiques (25) sont des séquences consensus minimales requises pour qu'une molécule d'ARN soit capable d'hydrolyser une autre molécule d'ARN suivant un mode catalytique. Ces ribonucléotides à activité catalytique (ribozymes) sont capables de cliver un ARN cible sur lequel ils sont hybridés de manière spécifique (grâce à deux séquences de 15 nucléotides complémentaires à l'ARN cible et placées de part et d'autre de la séquence catalytique). Ces séquences ribozymiques comparables à de "super antisens" peuvent être utilisées avec profit dans notre système en augmentant l'efficacité fonctionnelle de l'ensemble.

Par ailleurs, le gène VA est de petite taille (160 nucléotides pour VA I de l'adénovirus 2). Cela permet selon la présente invention d'utiliser de manière simultanée plusieurs gènes VA-antisens portés par une même construction génétique. Le but recherché est de définir des "cocktails" antisens, (par exemple dans le cas du VIH utilisation concomitante de trois gènes différents; anti-rev, anti-tat et anti-signal d'encapsidation). Ces constructions génétiques multiples présentent deux avantages importants :

- additivité des gènes et donc augmentation de la concentration intracellulaire des ARN antisens.
- multiplicité des séquences cibles et donc meilleure efficacité de fonctionnement.

De plus, dans le cas de virus dotés d'une forte variabilité génétique et qui "s'adaptent" au traitement utilisé, le recours à l'utilisation de plusieurs séquences antisens permet d'éviter l'emergence de variants génétiques.

La présente invention a donc aussi pour objet un vecteur comportant plusieurs gènes viraux identiques ou différents transcrits par l'ARN polymérase III dans lequel on a inséré un oligonucléotide identique ou différent en dehors des boites A et B de chacun desdits gènes viraux.

La présente invention a également pour objet un procédé de production intracellulaire d'un fragment d'ARN in vitro dans lequel on transfecte ou infecte des cellules eucaryotes comportant de l'ARN polymérase III avec un vecteur réplicatif selon l'invention, comportant comme oligonucléotide un fragment d'ADN correspondant au transcrit inverse dudit ARN et en ce que l'on cultive dans un milieu de culture approprié les cellules eucaryotes ainsi transfectées ou infectées.

La présente invention a en outre également pour objet, comme on l'a vu, l'utilisation à titre de médicament, d'un vecteur selon l'invention, dans lequel l'oligonucléotide est transcrit en une molécule ARN "antisens" ou une molécule d'ARN ribozymique bloquant l'expression d'un gène impliqué dans une pathologie en s'hybridant et, le cas échéant, en coupant son ARN messager d'origine cellulaire virale, bactérienne ou parasitaire.

Le médicament selon l'invention peut être utilisé comme agent antiviral, antitumoral, antibiotique ou antiparasitaire, ou dans toute pathologie où un gène est anormalement exprimé soit par mutation, soit par dérégulation.

La présente invention a également pour objet un procédé de blocage de l'expression d'un gène ex vivo à l'aide d'un vecteur selon l'invention dont ledit oligonucléotide est transcrit en une molécule ARN antisens ou ribozymique qui s'hybride à, ou respectivement, coupe l'ARN messager dudit gène.

La présente invention a aussi comme objet un procédé de traitement de cellules ex vivo à l'aide d'un vecteur selon l'invention.

Dans ses applications thérapeutiques ex-vivo, on utilise de façon tout à fait appropriée un vecteur viral ou rétroviral qui pénètre dans la cellule par transfection ou infection. En particulier à titre de médicament selon l'invention, on utilise un vecteur constitué par un vecteur viral défectif tel qu'un adénovirus ou un vecteur rétroviral défectif tel qu'un rétrovirus murin.

En effet, le vecteur utilisé pour véhiculer la construction génique selon l'invention vers sa cible théorique, peut être un vecteur rétroviral avec transport de la construction recombinante par une capside d'emprunt et insertion du matériel génétique dans le DNA de la cellule hôte.

Les techniques consistant à utiliser des vecteurs, notamment viraux, pour transporter et faire pénétrer du matériel génétique dans des cellules cibles et introduire de manière efficace des modifications génétiques dans divers tissus somatiques comme le muscle, le foie, le cerveau et les cellules hématopoïétiques sont connues de l'homme de l'Art. En particulier, le tissu hématopoïétique (leucocytes, hématies, plaquettes...) présente deux caractéristiques essentielles qui font de ce tissu un bon candidat pour les approches de thérapie génique :

- les cellules sanguines sont aisément prélevables sans traumatisme pour le patient. De plus, les conditions de culture de ces cellules (utilisation de diverses cytokines) se sont affinées et permettent un maintien ex vivo pour des périodes de temps variables (congélation, culture).
- l'étude de la différenciation des différentes lignées composant le système hématopoïétique a permis de montrer que l'ensemble de ces lignées dérivent d'un progéniteur commun (Uchida N., Fleming W. H., Alpern E.J. and Weissman I.L. 1993 Current Opinion in Immunology, 5, 177-184). Cela permet d'introduire la modification génétique souhaitée dans des cellules souches qui, après réimplantation, sont capables de recoloniser en totalité le tissu hématopoïétique.

La caractérisation des cellules précurseurs a permis d'établir que la présence d'une protéine de surface (CD34) permet de les distinguer des autres types cellulaires comme étant des cellules CD34+.

A tous les stades de la différenciation, les cellules hématopoïétiques prolifèrent. De ce fait, l'introduction de gènes étrangers dans ces cellules impose que celui-ci ne soit pas "dilué" au cours des divisions cellulaires successives. Les rétrovirus, qui s'intègrent définitivement dans le génome de la cellule récipiente et pour lesquels le mécanisme moléculaire de réplication est relativement bien connu, apparaissent comme de bons candidats pour la thérapie génique des cellules hématopoïétiques.

L'utilisation des vecteurs rétroviraux pour transporter du matériel génétique nécessite, d'une part, de réaliser la construction génétique du rétrovirus recombinant, et d'autre part, de disposer d'un système cellulaire qui assure la fonction d'encapsidation du matériel génétique à transporter :

- Dans un premier temps, les techniques de génie génétique permettent de modifier le génome d'un rétrovirus murin comme le virus de Moloney (rétrovirus murin appartenant au groupe des virus des leucémies murines : Reddy E.P., Smith M. J. and Aaronson S.A., 1981, Science. 214, 445-450). Le génome rétroviral est cloné dans un vecteur plasmidique puis délété de l'ensemble des séquences virales codant pour les protéines de structure (gènes : Gag, Env) ainsi que la séquence codant pour les activités enzymatiques (gène ; Pol). De ce fait, seules les séquences nécessaires "en cis" pour la réplication, la transcription et l'intégration sont conservées (séquences correspondants aux deux régions LTR, signal d'encapsidation et signal de fixation de l'amorce). Les séquences génétiques délétées peuvent être remplacées par des gènes non viraux comme le gène de résistance à la néomycine (antibiotique de séléction pour les cellules eucaryotes) et par le gène à transporter par le vecteur rétroviral.

- Dans un second temps, la construction plasmidique ainsi obtenue est introduite par transfection dans les cellules d'encapsidation. Ces cellules expriment de manière constitutive les protéines virales Gag, Pol et Env, mais l'ARN codant pour ces protéines est dépourvu des signaux nécessaires à son encapsidation. De ce fait, cet ARN ne peut pas être encapsidé et permettre la formation de particules virales. Seul l'ARN recombinant, issu de la construction rétrovirale transfectée, est dotée du signal d'encapsidation et est encapsidée. Les particules rétrovirales produites par ce système contiennent l'ensemble des éléments nécessaires pour l'infection des cellules cibles (telles que les cellules CD34+) et pour l'intégration définitive du gène d'intérêt dans ces cellules. L'absence des gènes Gag, Pol, Env empêche le système de continuer à se propager.

Selon la présente invention les gènes VA-antisens ont comme caractéristique d'être transcrit par l'ARN Polymérase III. Cette particularité a conduit à développer deux types de constructions rétrovirales dans lesquelles les gènes VA-anti rev ont été clonés comme décrit à l'exemple 6.

Les virus à ADN, tels que les adénovirus, peuvent également convenir à cette approche, bien que, dans ce cas, le maintien du DNA à l'état épisomique sous forme de réplicon autonome soit la situation la plus probable.

Il va de soi que l'utilisation d'un vecteur viral, provenant d'un Adénovirus, est particulièrement adaptée lorsque le gène viral est un gène d'Adénovirus. Les adénovirus présentent certaines propriétés intérressantes. Notamment, ils ont un spectre d'hôte assez large, sont capables d'infecter des cellules quiescentes, et ils ne s'intègrent pas au génome de la cellule infectée. Pour ces raisons, les adénovirus ont déjà été utilisés pour le transfert de gènes in vivo. A cet effet, différents vecteurs dérivés des adénovirus ont été préparés, incorporant différents gènes (bêta-gal, OTC, alpha-1At, cytokines, etc). Pour limiter les risques de multiplication et la formation de particules infectieuses in vivo, les adénovirus utilisés sont généralement modifiés de manière à les rendre incapables de réplication dans la cellule infectée.

Ainsi, les adénovirus utilisés sont généralement délétés des régions E1 (E1a et/ou E1b) et éventuellement E3.

Les adénovirus recombinants défectifs selon l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol 36 (1977) 59) qui contient notamment, intégrée dans son génome, la partie gauche du génome d'un Adénovirus Ad5 (12%).

Ensuite les adénovirus qui se sont multipliés sont récupérés et purifiés selon les techniques classiques de biologie moléculaire.

Selon la présente invention, dans le génome de l'adénovirus recombinant défectif est insérée, au niveau du gène VA, une séquence d'ADN exogène notamment codant pour un ARN antisens.

Les compositions pharmaceutiques comprenant un ou plusieurs vecteur virus tels que des recombinants déféctifs comme décrits précédemment peuvent être formulées en vue d'une administration par voie topique, orale, parentérale, intranasale, intraveineuse, intramusculaire, sous-cutanée, intraoculaire, etc. Préférentiellement, elles contiennent des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels) stériles, isotoniques, ou de composition sèches, notamment lyophilisées, qui, par addition selon le cas d'eau stérilisée ou de sérum physiologique, permettent la constitution de solutés injectables particuliers de solutions stériles, isotoniques, ou de compositions sèches, notamment lyophilisées, qui par addition selon le cas d'eau stérilisé&e ou de sérum physiologique, permettent la constitution de solutés injectables.

Les doses de virus recombinant défectif utilisés pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les virus recombinants selon l'invention peuvent être formulés et administrés sous forme de doses comprises entre 10 et 10 pfu/ml, et de préférence 10 à 10 pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

L'utilisation de virus modifiés génétiquement comme système navette pour transporter le matériel génétique modifié permet non seulement de faire pénétrer le matériel génétique dans la cellule récipiente par le biais de l'utilisation d'une capside virale d'emprunt, mais permet également de traiter de manière simultanée, et sur une courte période de temps, un grand nombre de cellules ; ceci ouvre la voie à des approches curatives s'adressant in vitro à des cellules déjà infectées par le virus à inhiber, mais permet également le traitement thérapeutique appliqué à l'organisme entier.

Selon l'invention, on peut utiliser des transactivateurs viraux. En particulier, la protéine Ela d'Adénovirus stimule l'activité transcriptionnelle de la polymérase III (23), notamment en mobilisant le facteur TFIIIC limitant. Cet effet potentialisateur de la polymérase III est surtout observé si l'ADN porteur du gène VA est sous forme épisomique (cas fréquent pour les Adénovirus). On peut utiliser cette propriété de la protéine E1A pour stimuler l'efficacité du système "VA-ARN polymérase III" surtout au cours d'expériences d'expression transitoire, soit avec le gène E1a cloné en cis du gène VA-ARN, soit en trans par cotransfection.

Egalement, la protéine Tat du HIV pourrait stimuler l'activité transcriptionnelle de la polymérase III.

L'utilisation des transactivateurs viraux couplée avec la vectorisation de l'antisens peut être mise à profit selon l'invention, en matière de spécifité d'action vis à vis de cellules infectées par le HIV (ou d'autres virus pathogènes). Deux stratégies complémentaires peuvent être appliquées:

- Dans le but de conférer une "immunité cellulaire" à des cellules permissives à l'infection HIV (CD4+), le traitement est réalisé "ex-vivo" par le prélèvement de cellules souches issues de la moelle, leur tri et leur modification génétique "ex-vivo" ;
- Une stratégie "curative" est également possible grâce à l'instauration d'une dépendance de la réplication du vecteur pour la présence de la protéine Tat du HIV. Cette dépendance permet de répliquer efficacement le vecteur uniquement dans des cellules infectées par le virus.

La mise au point et le développement de cassettes antisens selon l'invention, adaptables à de nombreux sujets de recherche sont envisagés dans de très nombreux domaines dont notamment l'oncologie moléculaire, le déterminisme cellulaire.

D'autres caractéristiques et avantages de la présente invention apparaîtront à la lumière de la description détaillée du mode de réalisation suivant.

La Figure 1 représente la séquence nucléotidique du gène VAI de l'Adénovirus 2.

LEGENDE de la figure 1:

| | |
|---|---|
| XXXXXX | Gène VAI-ARN (Région transcrite) |
| XXXXXX | Oligonucléotides en 5'et 3' du gène VAI servant d'amorces pour la P.C.R. |
| XXXXXX | Séquences des Box A et B nécessaires à la transcrition du gène VAI-ARN par la polymérase III |
| xxxxxxx | Site d'insertion de séquences exogènes |

La Figure 2 représente des séquences antisens et random insérées au nucléotide 10711 du gène VAI.

La Figure 3 représente l'analyse sur gel d'acrylamide/urée des produits de transcription acellulaire des constructions PVV2/VAI/antisens.

La Figure 4 représente la cinétique d'infection par HIV de CEM transfectées ou non par un antisens.

La Figure 5 représente le dosage des antigènes HIV présents dans les surnageants des cellules CEM transfectées par des constructions antisens.

Les dosages d'antigènes HIV ont été effectués 5 heures après renouvellement du surnageant de culture et 24 jours après le début de l'infection.

Des structures secondaires des VA-ARN : la structure VA-ARNI (A) et deux structures possibles pour les VA-ARNII (B et C) sont décrits dans la référence 13.

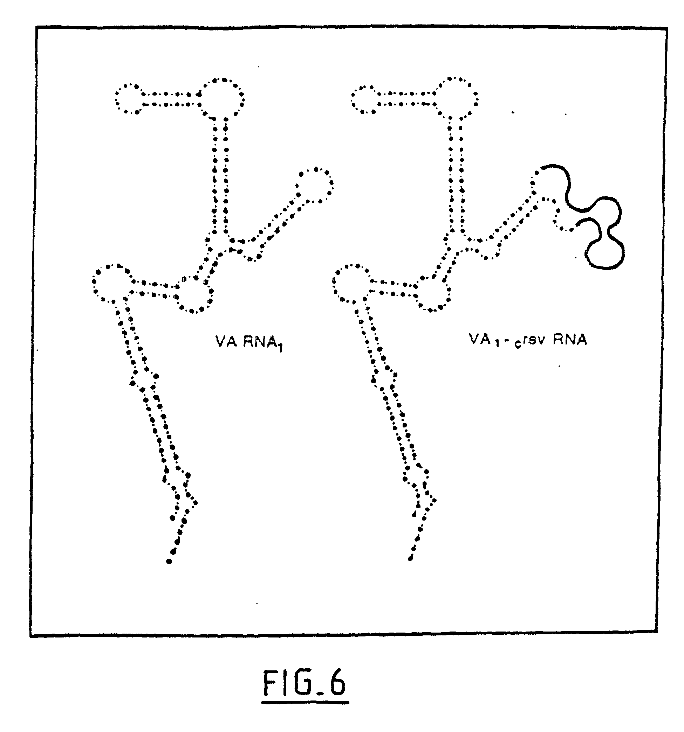

La figure 6 représente la structure secondaire du gène VA I.

La Figure 7 représente le schéma d'insertion d'un oligonucléotide par méthode PCR "overlap".

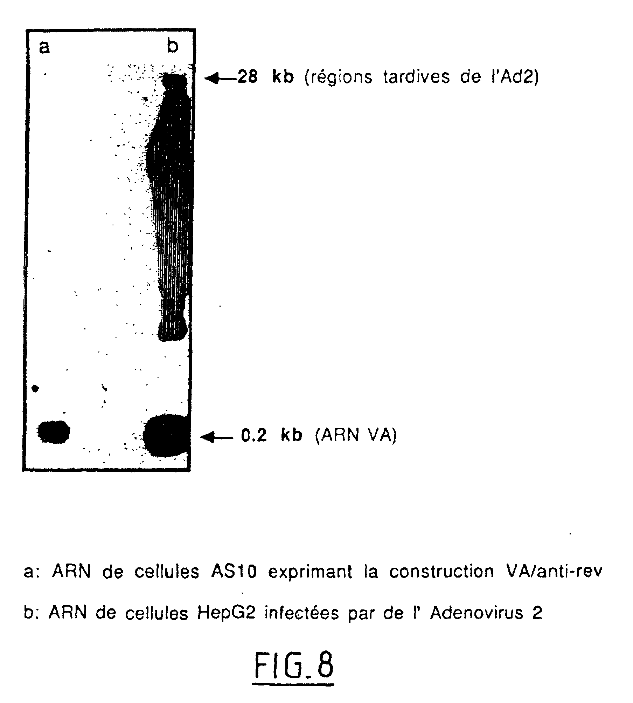

La Figure 8 représente l'expression du VA-antisens dans la population $AS_{10}$

piste a : ARN de cellules de la population $AS_{10}$
piste b : ARN de cellules de la lignée $HepG_2$ infectée par l'Adénovirus de type 2.

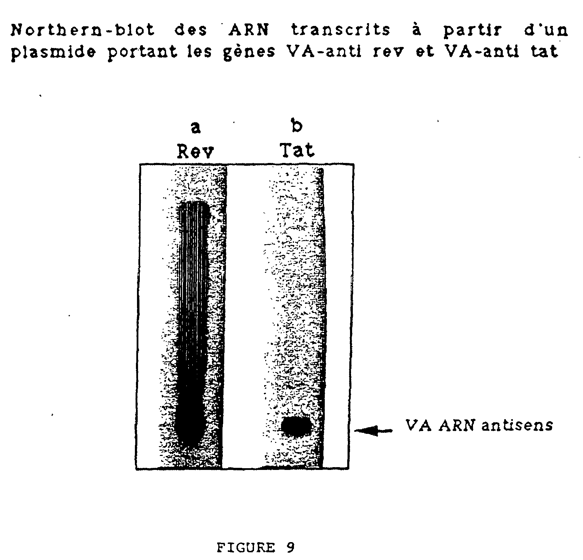

La figure 9 présente des résultats de Northern-Blot des ARN transcrits à partir d'un plasmide portant les gènes VA-anti rev et VA-anti rat obtenus à l'exemple 5 avec la construction portant le gène rev et le gène tat.

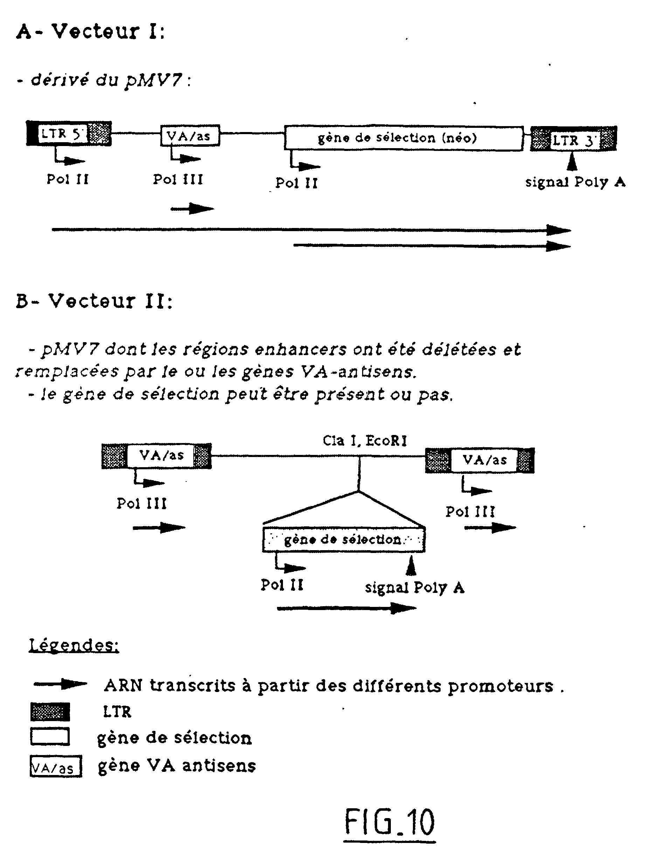

La figure 10 représente la construction des vecteurs rétroviraux I et II dérivés du vecteur pMV7 de l'exemple 6.

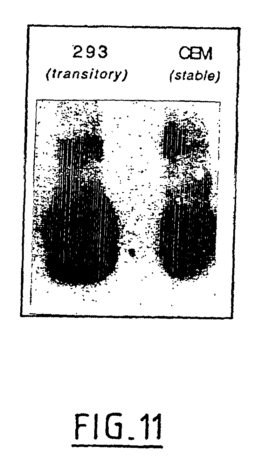

La figure 11 représente l'expression transitoire des gènes VA I modifiés de l'exemple 7 dans des cellules 293.

### Exemple 1 : Clonage du gène VAI

Le gène choisi pour les expériences proposées ci-après est le gène VA-ARNI de l'Adénovirus 2. La séquence de l'Adénovirus est disponibles dans la banque de données : Genbank - réf.: ADBVAI, séquences transcrites du nucléotide 10610 au nucléotide 10769 (la figure 1 présente les éléments de séquence concernés). La conformation de l'ARN en solution est publiée et présentée dans la Figure 6 (13,14).

Le clonage du gène VAI recombinant est réalisé dans un vecteur plasmidique de type PVV2 (15) porteur en cis du gène de résistance à la généticine (sous dépendance du promoteur TK) ou dans un vecteur à réplication autonome en système eucaryote du type pCEP-4 (commercialisé par Invitrogen) ou encore pHEBo (aimablement fourni par R.P. SEKALY, laboratoire d'Immunologie, Montréal). Ces derniers vecteurs sont porteurs des régions OriP et Ebna-1 et ils portent également le gène de résistance à l'hygromycine (16,17).

Le gène VAI de l'Adénovirus 2 a été cloné après une étape d'amplification par PCR. Les oligonucléotides servant à cette PCR ont été choisis du coté 5' en amont du site de début de transcription du gène VAI et du coté 3' en aval du site TTTT de terminaison (Fig. 1). Les extrémités 5' de chaque oligonucléotide sont dotées du site de coupure enzymatique ClaI, site unique sur le plasmide pVV2, ou bien du site HindIII pour le clonage sur le plasmide pHEBo. La transformation de bactéries par des constructions "pVV2-VAI" a permis d'obtenir plusieurs clones recombinants.

### Exemple 2 : Insertion et clonage des séquences antisens

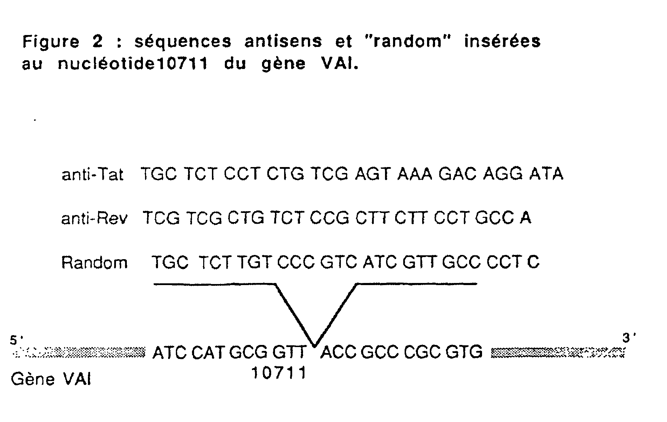

Pour le HIV, la séquence des oligonucléotides à insérer a été déterminée dans un premier temps conformément aux séquences antisens déjà publiées par d'autres auteurs : anti-rev (18) ou anti-tat (19) et séquence aléatoire servant de contrôle spécifique (voir Fig. 2) (les protéines tat et rev sont deux protéines virales dont le rôle régulateur est critique pour la réplication du virus).

Le recours à des séquences de type ribozyme, est actuellement en cours, avec clonage des recombinants porteurs de séquences ribozymiques publiées par Goodchild & Kohli en 1991 (20).

L'utilisation de la PCR "overlap" (voir Figure 7) a permis d'insérer des oligonucléotides de taille variable dans le gène VAI. Les antisens "anti-rev", "anti-tat" et "random" ont été insérés au niveau du nucléotide 10711 de la séquence du VA-ARNI (Fig. 2). Les gènes VAI ainsi modifiés par l'insertion de la séquence antisens sont clonés dans le site ClaI du plasmide pVV2 comme décrit précédemment.

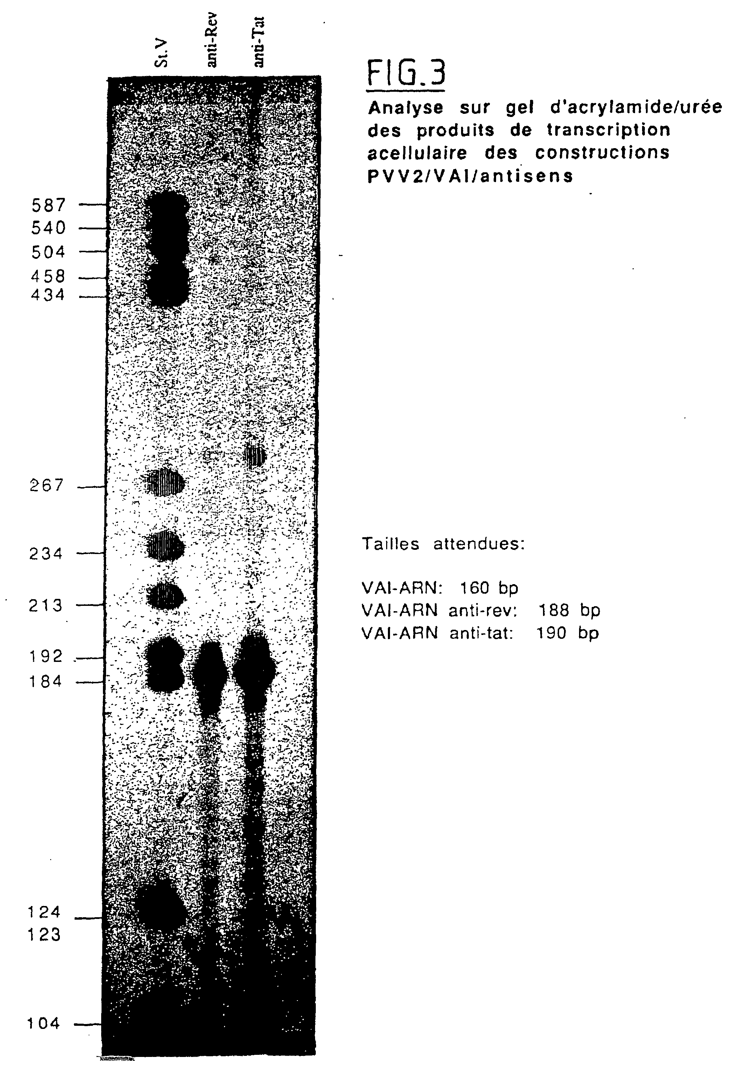

Les constructions obtenues ont toutes été séquencées (sur 200 nucléotides) puis testées en système de transcription acellulaire pour s'assurer de la fonctionnalité ainsi que de l'efficacité relative de transcription de chaque construction. Pour la mise au point des transcriptions in vitro, le protocole suivi est celui décrit par Wu (21) et Weil (22). Brièvement, $3\mu g$ d'ADN à tester sont incubés 90 min à 29°C en présence d'extraits cellulaires contenant l'activité polymérase III et de nucléotides dont notamment de alpha-$P^{32}$-dGTP. Après synthèse, les produits de la réaction sont analysés sur gel d'acrylamide (la Figure 3 présente le type de résultats obtenus). Les tailles observées correspondent bien aux tailles attendues pour chaque construction, à savoir : VA-ARNI natif 160 nucléotides, VA-ARNI/anti-rev 188, VA-ARNI/anti-tat 190. Les autres constructions VA-ARNI/ribozymes ne sont pas encore analysées.

Il est bien vérifié que l'insertion de séquence exogènes dans le gène VA n'affecte pas son taux de transcrition par la Polymérase III.

**Exemple 3 : Inhibition de la réplication virale**

La mise au point et le développement des outils antisens sont réalisés, dans le cas d'antisens antiviraux, sur les lignées cellulaires permissives pour la réplication du virus étudié. Par exemple, pour les antisens anti-HIV, les lignées lymphoblastoîdes CEM ou MOLT-4, toutes deux bonnes productrices de virus, sont choisies.

Les cellules de la lignée CEM (débarrassées de mycoplasmes) ont été transfectées par les différents plasmides recombinants (pVV2/VAI/anti-rev, pVV2/VAI/anti-tat, pVV2/VAI et pVV2 témoin. Les techniques de transfection cellulaire utilisées dépendent du modèle cellulaire étudié.

Pour les cellules en suspension, on a utilisé préférentiellement l'électroporation (Appareil de type Gene Pulser$^{(R)}$, Biorad, les chocs électriques sont produits à 200V et 960 µF).

Après transfection, les cellules transduites sont réparties dans 96 puits de culture indépendants et sélectionnées par action des antibiotiques généticine 1.5 mg/ml (plasmide pVV2) ou hygromycine 400 µg/ml (plasmide pHEBo). Le maintien de la pression de sélection reste nécessaire tout au long des expériences. A ce jour, en utilisant l'électroporation, la lignée CEM a été transfectée par des constructions recombinantes du plasmide pVV2/anti-rev, pVV2/anti-tat, pVV2/VAI et pVV2.

Des populations cellulaires non clonales, résistantes à la généticine, issues de puits de culture différents, sont sélectionnées sur la base d'une bonne multiplication cellulaire avec un faible taux de mortalité. Les populations cellulaires issues de la transfection avec des plasmides "anti-sens" sont numérotées AS indice X (AS : AntiSens, X n° du puits de sélection).

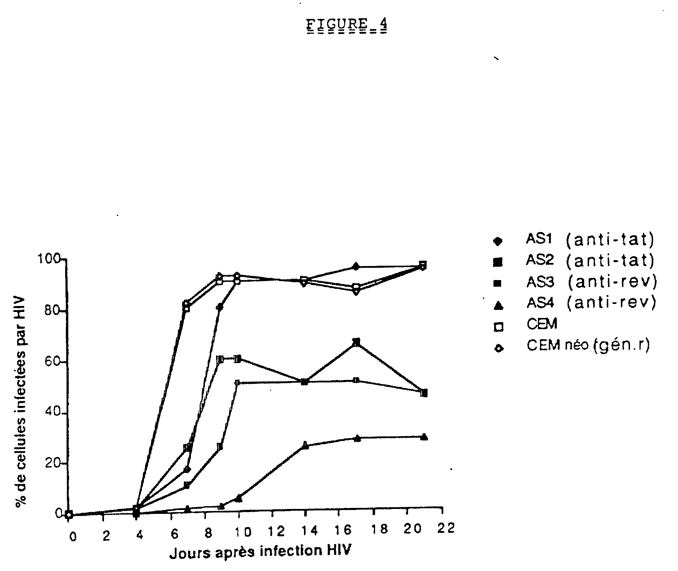

Chaque population est infectée par du surnageant infectieux provenant de cellules chroniquement infectées par le virus HIV (surnageant riche en particules), la souche utilisée ne présente pas d'effet cytopathique sur les cellules utilisées (souche LAV/BRU, JC Cherman, Marseille). La cinétique de réplication virale montre que, dans les conditions habituelles, plus de 95% des CEM produisent des particules virales 7 à 8 jours après l'infection (Figure 4).

La mesure de la réplication virale est estimée par immunofluorescence indirecte. Brièvement, 5000 cellules sont fixées sur lames de verre et incubées 30 min. en présence d'un sérum anti-HIV provenant d'un patient séropositif dilué au 1/40. La révélation est effectuée grâce à l'utilisation d'un deuxième anticorps, spécifique pour les immunoglobines humaines, conjugué à l'isothiocyanate de fluorescéïne (FITC).

La réplication virale peut être également mesurée par le dosage de la concentration d'antigènes HIV-1 présents dans le surnageant de culture (dosage réalisé avec le kit HIVAG-1$^{(R)}$ de ABBOT). Les cinétiques d'infection sont réalisées de manière simultanée sur les cellules transfectées par les différents plasmides et issues de la sélection par la généticine.

La Figure 4 présente les résultats obtenus en infectant de manière comparative la lignée CEM servant de témoin positif, la lignée CEM résistante à la généticine (CEM.gén.r.) transfectée par le pVV2), 2 populations "anti-tat" (AS1 et AS2) et 2 populations "anti-rev" (AS3 et AS4). La mesure de la réplication virale a été suivie par l'observation et le comptage du nombre de cellules positives HIV détectés par immunofluorescence indirecte à différents jours après l'infection :

La superposition des courbes "CEM" et "CEM gén.r." montre que la présence de généticine dans le milieu n'affecte pas l'infection des cellules CEM par le virus HIV. Il apparaît que les 4 populations "antisens" testées présentent un retard significatif pour le démarrage de l'infection, la période d'éclipse est prolongée de 2 jours pour AS1 et jusqu'à 6 jours dans le cas de AS3.

De plus, un faible pourcentage de cellules sont infectées pour certaines lignées (AS2, AS3, AS4) (25 à 60%) même tardivement après l'infection traduisant la résistance à l'infection virale d'un grand nombre de cellules au sein de ces populations non clonées. Ceci n'est pas le cas de la lignée AS1 qui présente plus de 95% de cellules positives 10 jours après le début de l'infection.

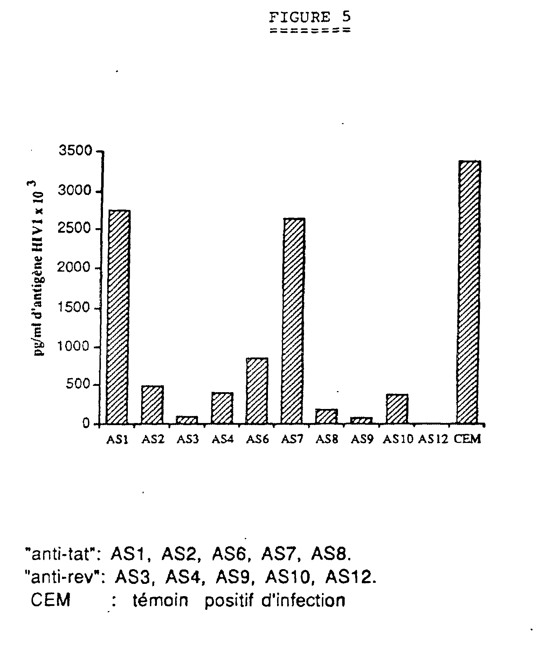

La Figure 5 présente les résultats obtenus au cours d'une expérience du même type, mais réalisée avec un pannel plus large de populations ASX et quantifiée par la mesure des antigènes HIV-1 présents dans les surnageants à différents jours après l'infection par HIV. Seuls sont présentés les résultats obtenus au jour 24 (ceux-ci étant représentatifs des résultats obtenus aux autres jours). Afin d'éviter l'accumulation des particules virales et de fausser les mesures comparatives, les surnageants sont collectés 5 heures après le changement de milieu précédent.

Les populations "VAI/anti-tat" correspondent aux conditions AS1, AS2, AS6, AS7 et AS8, et les populations "VAI/anti-rev" aux conditions AS3, AS4, AS9, AS10 et AS12.

Il apparaît que la plupart des populations "antisens" présentent une diminution significative de la quantité d'antigènes HIV-1 mesurée dans les surnageants, pour 8 lignées sur 10 (AS2, AS3, AS4, AS6, AS8, AS9, AS10 et AS12), on mesure moins de 20% de production d'antigènes viraux comparativement au contrôle, 2 populations atteignent 70% du niveau contrôle (AS1 et AS7).

Les résultats obtenus pour les populations AS1,2,3 et AS4 selon 2 techniques de mesure de la réplication virale : Immunofluorescence (Figure 4) et concentrations en antigènes (Figure 5) sont concordants. Dans les Figures 4 et 5, les populations AS1, AS2, AS3 et AS4 sont identiques.

## Exemple 4 : Mesure du taux d'expression du VA-ARNI

Il est possible de caractériser l'expression des ARN antisens grâce aux techniques de Northen-blot ou bien d'hybridation en milieu liquide. Les ARN sont préparés suivant le protocole suivant : $25.10^6$ sont rincées deux fois par du tampon salin, les cellules sont lysées à 4°C par choc hypotonique (Tris 10 mM ph 7,4, NaCl 10 m$^M$, MgCl$_2$, 3 mM) et par action de NP 40 1% ; après centrifugation (10.000 rpm, 10 minutes), le surnageant contenant les ARN cytoplasmiques est débarrassé des protéines résiduelles par 3 extractions au phénol-chloroforme et les ARN sont concentrés à l'éthanol. 10 µg d'ARN sont analysés par Northern-blot et hybridés avec une sonde monobrin spécifique de la région 3' du gène VA. Les résultats obtenus sont présentés sur la Figure 8. Les résultats présentés ici illustrent le fait que in vivo les constructions testées (ici A5$_{10}$, "VA/anti-rev") sont exprimées et que le produit de transcription obtenu a bien la taille attendue.

## Exemple 5 : Utilisation simultanée de plusieurs gènes VA-chimères : "Cocktails-antisens"

On a réalisé une construction génétique comportant la succession des deux gènes VA-anti rev et VA-anti tat, clonés respectivement dans les sites Cla1 et Hind III du plasmide pVV2. Des expériences de transcription acellulaires, réalisées avec cette construction, ont démontré que chacun de ces gènes s'exprimait correctement :

Les ARN, issus de la transcription en système acellulaire du plasmide portant les deux gènes, ont été analysés par Northern-blot et révélés soit avec une sonde spécifique de la séquence rev, soit avec une sonde spécifique de la séquence tat. Les résultats obtenus sont présentés sur la figure 9.

- Le protocole de transcription acellulaire est celui déjà utilisé à l'exemple 2 et pour la figure 3 (ref 21 WU et al, et 22 Weil et al)
- L'analyse des ARN par Northern-blot est réalisé après purification des ARN (traitement par la DNase I puis la protéinase K et extraction au phénol-chloroforme), séparation sur gel d'agarose en milieu dénaturant, transfert sur membrane et révélation soit avec la sonde rev (piste a) soit avec la sonde tat (piste b).

## Exemple 6 : Vectorisation des gènes VA-antisens par des rétrovirus murins modifiés - Transfert aux progéniteurs des cellules hématopoîétiques.

1) Vecteur rétroviral de type I :

Le vecteur qui est utilisé est le plasmide pMV7 (P.T. Kirschmeier, G.M. Housey, M.D. Jonhson, A.S. Perkins and I.B. Weinstein, 1988, DNA, Vol. 7, 3, 219-225). Il est composé d'une part des séquences nécessaires à la maintenance plasmidique et d'autre art des deux LTR du virus Moloney encadrant le gène de résistance à la néomycine (sous la dépendance du proimoteur TK) . Les sites de clonage Hind III, ClaI et EcoRI situés entre le LTR 5' et le gène néomycine permettent l'insertion de séquences exogènes. La cellule d'encapsidation est la cellule DAMP (R. Mann, R.C. Mulligan and D. Baltimore, 1983, Cell, 33, 143-159). On a cloné les constructions génétiques au site Hind III du pMV7 et transfecté les plasmides recombinants obtenus dans la cellule DAMP. Les cellules DAMP devenues résistantes à la néomycine ont été sous-clonées. Les clones présentant un fort pouvoir infectieux, détecté dans les surnageants de culture, ont été sélectionés. Ces clones ont permis d'infecter des lymphocytes de la lignée CEM (lymphocytes T4 auxilliaires). L'infection est réalisée grâce à une co-culture d'une nuit, entre les DAMP et les lymphocytes. Les lymphocytes ayant été infectés sont devenus, à leur tour, résistants à la néomycine et peu-

vent être ainsi sélectionné. Les ARN provenant de ces lymphocytes ont été analysés et l'expression du gène VA-anti rev (porté par la séquence provirale intégrée) détectée.

Cependant, l'interférence possible entre l'activité de l'ARN Polymérase II qui transcrit la totalité du génome viral à partir du LTR 5' et l'activité de l'ARN Polymérase III pourrait, dans certains cas, se révéler être un élément défavorable pour l'efficacité d'un tel système.

2) Vecteurs rétroviraux de type II :

On a délété les signaux enhancers présents dans la région U3 du LTR 3' du virus Moloney cloné dans le pMV7 et on a remplacé ces signaux par des sites de clonage unique sur ce plasmide (ces modifications étant réalisées grâce à la technologie PCR). Les constructions antisens sont insérées à ces sites. Cette modification présente deux avantages : elle permet d'une part de dupliquer le gène cloné grâce au jeu de la reverse transcription (la région U3 présente sur chacun des deux LTR provient de la région U3 située sur le LTR en 3' du provirus précédent) mais surtout, cela ne permet plus à l'ARN Polymérase II de transcrire le provirus intégré dans la cellule récipiente. Cela évite l'interférence avec l'ARN Polymérase III et assure une sécurité de fonctionnement vis à vis de l'activation toujours possible de gènes adjacents.

Plus précisément, le vecteur II a été obtenu comme illustré figure 10:

1) le site unique Hind III a été délété (coupure, remplissage avec la DNA polymérase, religature).

2) la digestion complète du pMV7 par Cla I libère le gène de sélection (néomycine), la pârtie plasmidique résultante est ensuite traitée par l'enzyme Xho I de manière partielle. Seul le fragment contenant le LTR 5' est purifié sur gel d'agarose (5064 pb). De ce fait, le LTR 3' est perdu.

3) le LTR 3' est ensuite remplacé par un LTR 3' modifié pour lequel les régions enhancers situées dans la région U3 ont été supprimées et remplacées par un site Hind III (ceci est réalisé grâce à la technologie P.C.R.).

4) le gène antisens a été cloné dans le site Hind III nouvellement créé.

5) cette nouvelle construction rétrovirale peut être utilisée de manière habituelle :

- transfection dans des cellules d'encapsidation (cellules CRIP ref Danos and R.C. Mulligan, Proc. Natl. Acad. Sci., 1988, vol. 85, 6460-6446).
- récupération des surnageants avec estimation du titre infectieux.
- ces particules rétrovirales recombinantes seront utilisées pour transduire les cellules cibles.

Dans le but d'éviter l'obtention de particules recombinantes sauvages du virus Moloney, la cellule d'encapsidation DAMP est changée au profit de la cellule CRIP (O. Danos and R.C. Mulligan, Proc. Natl. Acad. Sci., 1988, 85, 6460-6446). La cellule CRIP contient deux génomes rétroviraux auxiliaires délétés de l'extrémité LTR 3', du signal d'encapsidation et mutés l'un dans la région Gag, l'autre dans la région env.

Par complémentation la production des protéines Gag, Pol et Env est assurée mais la probabilité d'obtention de génomes sauvages obtenus par recombinaison est réduite à zéro. L'ensemble des améliorations proposées et décrites ici permet d'assurer la production de particules rétrovirales recombinantes portant un ou plusieurs gènes VA/antisens (pMV/AS) correctement transcrits par la Polymérase III. Ce vecteur offre également une sécurité optimale de fonctionnement en vue des applications thérapeutiques envisagées.

## EXEMPLE 7 : Expression transitoire des gènes VA-antisens

Dans la perspective d'une thérapeutique localisée à court terme, on a vérifié, en système cellulaire, l'expression transitoire des gènes VA-antisens. En expression transitoire, le gène VA-antisens n'est pas intégré dans le génome de la cellule hôte et s'accumule de façon importante dans le noyau où il est transcrit.

Des cellules adhérentes (lignée 293 : cellules embryonnaires de rein, humaines) ont été transfectées par 20 g du plasmide pVA anti rev. La technique utilisée est la transfection au phosphate de calcium. Cette technique consiste à mettre en contact pendant 18 heures, l'ADN plasmidique précipité par le phosphate de calcium et les cellules en augmentant l'absorption de l'ADN surt les membranes cellulaires et en limitant l'action des DNases cellulaires sur l'ADN entrant. Les ARN sont préparés 48 heures après la transfection, comme décrit précédemment, et analysés par Northern-blot avec une sonde spécifique des VA ARN anti rev. La figure 11 montre une expression transitoire des ARN (piste 293) plus forte en comparaison avec les ARN produits dans les cellules qui expriment de manière stable l'ARN VA intégré (piste CEM).

## REFERENCES

1. Weintraub H., Les ADN et les ARN antisens., (1990), Pour la science, 149: 54-61.

2. Melton D.A., Injected anti-sense RNAs specifically block messenger RNA translation in vivo., (1985), Proc. Natl. Acad. Sci. USA, **82**, 144-148.

3. Geiduschek E.P., Tocchini-Valentini G.P., Transcription by polymerase III, (1988), Ann. Rev. Biochem, **57**, 873-914.

4. Galli G., Hofstetter H., Birnstiel M.L., Two conserved sequence blocks within eukaryotic tRNA genes are major promoter elements., (1981), Nature **294**, 626-631.

5. Cotten M., Birnstiel M.L. Ribozyme mediated destruction of RNA in vivo., (1989), EMBO J., **8**, 3861-3866.

6. Vasseur M., Les virus oncogènes., (1989), Herman eds., Adenovirus, 151-184.

7. Furtado M.R., Subramanian S., Bhat R.A., Fowlkes D.M., Safer B., Thimmappaya B., Functional dissection of Adenovirus VAI RNA (1989), J. Virol., **63**, 3423-3434.

8. Thimmappaya B., Weinberger C., Schneider R. J., Shenk T., Adenovirus VAI RNA is required for efficient translation of viral mRNAs at late time after infection., (1982), Cell, **31**, 543-551.

9. Akusjärvi G., Svensson C., Nygard O., A mechanism by which adenovirus-associated RNAI controls translation in a transient expression assay., (1987), Mol. Cell. Biol., **7**, 549-551.

10. Mellits K.H., Kostura M., Matthews M.B., Interaction of Adenovirus VA RNA with the protein kinase DAI: Non equivalence of Binding and fonction., (1990), Cell, **61**, 812-815.

11. Ghadge G.D., Swaminanthan S., Katze M.G., Thimmappaya B., Binding of the adenovirus VAI RNA to the interferon-induced 68-kDa protein kinase correlates with function., (1991), Proc. Natl. Acad. Sci. USA, **88**, 7140-7144.

12. Jennings P.A., Molloy P.L., Inhibition of SV40 replicon function by engineered antisense RNA transcribed by RNA polymerase III., (1987), EMBO J., **6/3**, 3043-3047.

13. Akusjärvi G., Mathews M.B., Andersson P., Vennstrom B., Petterson U., Structure of genes for virus-associated RNA1 and RNA2 of adenovirus type 2., (1980), Proc. Natl. Acad. Sci. USA, 77/5, 2424-2428.

14. Monstein H.J., Philipson L., The conformation of adenovirus VAI RNA in solution, (1981), Nucleic Acids Res., **9**, 4239-4250.

15. Meneguzzi G., Binétruy B., Grisoni M., Cuzin F., Plasmidial maintenance in rodent fibroblasts of BPV1-pBR322 shuttle vector without immediately apparent oncogenic transformation of the recipient cells, (1984), EMBO J., **3**, 112-116.

16. Hambor J.E., Hauer C.A., Shu H.K. Groger R. K., Kaplan D.R., Tikoncinsky M.L., Use of an Epstein Barr virus episomal replicon for anti-sense RNA-mediated gene in a human cytotoxic T cell-clone., (1989), Proc. Natl. Acad. Sci. USA, **85**, 4010-4014.

17. Yates J.L., Warren N. Sugden B., Stable replication of plasmids derived from Epstein Barr virus in various mammalians cells., (1985), Nature, **313**, 812-815.

18. Matsukura M., Zon G., Shinozuka K. Robert-Guroff M., Shimada T., Stein C.A., Mitsuya H., Wong-Staal F., Cohen J.S. Broder S., Regulation of viral expression of human immunodeficiency virus in vitro by an antisense phosphorothioate oligodeoxynucleotide against rev (art/trs) in chronically infected cells., (1989), Proc. Natl. Acad. Sci. USA, **86**, 4244-4248.

19. Rhodes A., James W., Inhibition of human immunodeficiency virus replication in cell culture by endogenously synthesized antisense RNA., (1990), J. Gen. Virol., **71,** 1965-1974.

20. Sarver N., Cantin E.N., Chang P.S., Zaia J.A., Ladne P.L., Stephens D.A., Rossi J.J., Ribozymes as potential anti-HIV-1 therapeutic agents., (1990), Science, **247**, 1222-1225.

21. Wu G.-J., Zubay G., Prolonged transcription in a cell-free system involving nuclei and cytoplasm (1974), Proc. Natl. Acad. Sci. USA, **71**, 1803-1807.

22. Weil A.A., Segall J., Harris B., Ng S.-Y., Roeder R.G., Faithful transcription of eucaryotic genes by RNA polymerase III in systems reconstituted with purified DNA templates., (1979), J. Biol. Chem., **254**, 6163-6173.

23. Hoeffler W.K., Roeder R.G., Enhancement of RNA polymerase III transcription by the Ela gene product of adenovirus, (1985), Cell., **41**, 955-963.

24. Chomczyski P., Sacchi N., Single-Step Method of RNA isolation by Acid Guanidinium Thiocyanate-Phenol-Chloroform Extraction, (1987), Analytical Biochem., **162**, 955-963.

25. Haseloff J., Gerlach W.L., Simple RNA enzymes with new and highly specific endoribonuclease activities, (1988), Nature, **334**, 585-591.

26. Hambor J.E., Hauer C.A., Shu H.K., Groger R. K., Kaplan D.R., Tykocinski M.L., (1988), Proc. Natl. Acad. Sci. USA, **85**, 4010-4014.

27. Aufiero B., Schneider R.J., The hepatitis B virus X-gene product trans-activates both RNA polymerase II and III promoters, (1990), EMBO J., **9**, 497-504.

28. Danos O., Mulligan R., Safe and efficient generation of recombinant retroviruses with amphptropic and ectotropic host ranges, (1988), Proc. Natl. Acad. Sci. USA, **85**, 6460-6464.

29. Lévy J.P., Traitements du SIDA : recherche de nouveaux médicaments et élaboration de thérapies géniques, (1991), médecine/sciences, **7**, 830-841.

**Revendications**

1. Vecteur d'ADN recombinant comportant une cassette de transcription par l'ARN polymérase III, constituée par le gène VA I d'adénovirus transcrit par l'ARN polymérase III dans lequel on a inséré un oligonucléotide, fragment d'ADN, dans la région allant des nucléotides 10694 à 10730 dudit gène VA1 de l'Adénovirus 2 représenté Figure 1, ou à la place de la région allant des nucléotides 10694 à 10730, celle-ci étant délétée.

**2.** Vecteur selon la revendication 1, **caractérisé en ce que** ledit oligonucléotide est inséré dans la région allant des nucléotides 10702 à 10728 dudit gène VA1 de l'Adénovirus 2 représenté Figure 1, ou à la place de ladite région allant des nucléotides 10702 à 10728, celle-ci étant délétée, et le gène étant désigné VA delta IV.

**3.** Vecteur selon la revendication 1 ou 2, **caractérisé en ce que** l'oligonucléotide est inséré au nucléotide 10711 du gène VA-1 de l'Adénovirus 2 représenté Figure 1.

**4.** Vecteur selon l'une des revendications précédentes, **caractérisé en ce que** le vecteur est un vecteur réplicatif plasmidique, épisomique ou un vecteur viral.

**5.** Vecteur selon l'une des revendications précédentes, **caractérisé en ce que** le vecteur est un vecteur épisomique portant l'origine de réplication du virus Epstein Barr (oriP), ainsi que les séquences codantes pour la protéine EBNA 1.

**6.** Vecteur selon l'une des revendications précédentes, **caractérisé en ce qu'**il comporte plusieurs gènes viraux identiques ou différents transcrits par l'ARN polymérase III dans lesquels on a inséré un oligonucléotide identique ou différent en dehors des boites A et B dans chacun desdits gènes viraux.

**7.** Vecteur selon l'une des revendications précédentes, **caractérisé en ce que** l'oligonucléotide comporte de 15 à 40 nucléotides, de préférence 15 à 25.

**8.** Vecteur selon l'une des revendications précédentes, **caractérisé en ce que** le gène viral d'Adénovirus est un gène inactivé, par délétion ou mutation, en ce qui concerne l'inhibition de l'action de l'interféron.

**9.** Vecteur selon l'une des revendications précédentes, **caractérisé en ce que** l'oligonucléotide est inséré à l'intérieur de la région responsable de l'activité d'inhibition de l'action de l'interféron.

**10.** Vecteur selon l'une des revendications précédentes, **caractérisé en ce qu'**il s'agit d'un Adénovirus recombinant défectif.

**11.** Vecteur rétroviral à ARN, **caractérisé en ce qu'**il comporte le transcrit d'ARN de la cassette de transcription selon l'une des revendications 1 à 9.

**12.** Cellules infectées ou transfectées par un vecteur, selon l'une des revendications 1 à 11.

**13.** Procédé de production intracellulaire d'un fragment d'ARN in vitro dans lequel on transfecte ou infecte des cellules eucaryotes comportant de l'ARN polymérase III avec un vecteur selon l'une des revendications 1 à 11, comportant comme oligonucléotides un fragment d'ADN correspondant au transcrit inverse dudit ARN et en ce que l'on cultive dans un milieu de culture approprié les cellules eucaryotes ainsi transfectées ou infectées.

**14.** Procédé de blocage de l'expression d'un gène ex vivo à l'aide d'un vecteur selon l'une des revendications 1 à 11, dont ledit oligonucléotide est transcrit en une molécule ARN antisens ou ribozymique qui s'hybride et/ou coupe l'ARN messager dudit gène.

**15.** Procédé de traitement de cellules ex vivo à l'aide d'un vecteur selon l'une des revendications 1 à 11.

**16.** A titre de médicament, un vecteur selon l'une des revendications 1 à 11 utilisable notamment comme agent antiviral, antitumoral, antibiotique ou antiparasitaire, ou dans toute pathologie où un gène est anormalement exprimé soit par mutation, soit par dérégulation.

**17.** A titre de médicament, selon la revendication 16, un vecteur selon l'une des revendications 1 à 11, dans lequel l'oligonucléotide est transcrit en une molécule ARN "antisens" ou une molécule d'ARN ribozymique bloquant l'expression d'un gène impliqué dans une pathologie en s'hybridant et, le cas échéant, en coupant son ARN messager d'origine cellulaire virale, bactérienne ou parasitaire.

**18.** A titre de médicament selon la revendication 16 ou 17, un vecteur constitué par un vecteur viral défectif tel qu'un Adénovirus ou un vecteur rétroviral défectif tel qu'un rétrovirus murin.

**19.** A titre de médicament pour le traitement du SIDA, selon l'une des revendications 16 à 18, un vecteur selon l'une des revendications 1 à 11, comportant à titre d'oligonucléotides des séquences antisens notamment anti-rev ou anti-tat.

**Patentansprüche**

**1.** Rekombinanter DNA-Vektor, welcher eine Kassette für eine Transkription durch die RNA-Polymerase III umfasst, welche gebildet wird aus dem Gen VAI von Adenovirus, das durch die RNA-Polymerase III transkribiert wird, in welches man ein Oligonukleotid, ein DNA-Fragment, in der Region, die von Nukleotid 10694 bis Nukleotid 10730 des in Figur 1 dargestellten VAI-Gens von Adenovirus 2 geht, oder anstelle der Region, die von Nukleotid 10694 bis Nukleotid 10730 geht, wobei diese deletiert ist, insertiert hat.

2. Vektor nach Anspruch 1, **dadurch gekennzeichnet, dass** das Oligonukleotid in der Region, die von Mukleotid 10702 bis Nukleotid 10728 des in Figur 1 dargestellten VAI-Gens von Adenovirus 2 geht, oder anstelle dieser Region, die von Nukleotid 10702 bis Nukleotid 10728 geht, wobei diese deletiert ist, insertiert ist, und wobei das Gen als VA delta IV bezeichnet wird.

3. Vektor nach Anspruch 1 oder 2, **dadurch gekennzeichnet, dass** das Oligonukleotid bei Nukleotid 10711 des in Figur 1 dargestellten VA-1-Gens von Adenovirus 2 insertiert ist.

4. Vektor nach einem der vorangegangenen Ansprüche, **dadurch gekennzeichnet, dass** der Vektor ein plasmidischer, episomaler replikativer Vektor oder ein viraler Vektor ist.

5. Vektor nach einem der vorangegangenen Ansprüche, **dadurch gekennzeichnet, dass** der Vektor ein episomaler Vektor ist, der den Replikationsstartpunkt des Epstein Barr-Virus (oriP) sowie die das Protein EBNA 1 kodierenden Sequenzen umfasst.

6. Vektor nach einem der vorangegangenen Ansprüche, **dadurch gekennzeichnet, dass** er mehrere identische oder unterschiedliche virale Gene, die durch die RNA-Polymerase III transkribiert werden, umfasst, in welche man ein identisches oder unterschiedliches Oligonukleotid außerhalb der Boxen (Nukleotidsequenzabschnitte) A und B in jedem der genannten viralen Gene insertiert hat.

7. Vektor nach einem der vorangegangenen Ansprüche, **dadurch gekennzeichnet, dass** das Oligonukleotid 15 bis 40, vorzugsweise 15 bis 25 Nukleotide umfasst.

8. Vektor nach einem der vorangegangenen Ansprüche, **dadurch gekennzeichnet, dass** das virale Adenovirus-Gen ein Gen ist, welches durch Deletion oder Mutation hinsichtlich der Hemmung der Wirkung von Interferon inaktiviert worden ist.

9. Vektor nach einem der vorangegangenen Ansprüche, **dadurch gekennzeichnet, dass** das Oligonukleotid innerhalb der Region, die für die Aktivität der Hemmung der Wirkung von Interferon verantwortlich ist, insertiert ist.

10. Vektor nach einem der vorangegangenen Ansprüche, **dadurch gekennzeichnet, dass** es sich um ein rekombinantes defektes oder rekombinationsdefektes Adenovirus handelt.

11. Retroviraler RNA-Vektor, **dadurch gekennzeichnet, dass** er das RNA-Transkript der Transkriptionskassette nach einem der Ansprüche 1 bis 9 umfasst.

12. Durch einen Vektor nach einem der Ansprüche 1 bis 11 infizierte oder transfizierte Zellen.

13. Verfahren zur intrazellulären Herstellung eines RNA-Fragments in vitro, bei welchem man eukaryotische Zellen, die RNA-Polymerase III enthalten, mit einem Vektor nach einem der Ansprüche 1 bis 11, welcher als Oiigonukleotide ein DNA-Fragment, welches dem Umkehr- oder reversen Transkript der RNA entspricht, umfasst, transfiziert oder infiziert, und dass man in einem geeigneten Kulturmedium die so transfizierten oder infizierten eukaryotischen Zellen kultiviert.

14. Verfahren zur Blockierung der Expression eines Gens ex vivo mit Hilfe eines Vektors nach einem der Ansprüche 1 bis 11, von dem das Oligonukleotid in ein Antisinn- oder Ribozym-RNA-Molekül transkribiert wird, welches mit der mRNA des Gens hybridisiert und/oder diese spaltet.

15. Verfahren zur Behandlung von Zellen ex vivo mit Hilfe eines Vektors nach einem der Anspruche 1 bis 11.

16. Vektor nach einem der Ansprüche 1 bis 11 als Arzneimittel, welches insbesondere als antivirales Mittel, Antitumor-Mittel, antibiotisch oder antiparasitär wirksames Mittel oder bei einer jeglichen Pathologie, wo ein Gen entweder durch Mutation oder durch fehlerhafte Regulation in abnormaler Weise exprimiert wird, einsetzbar ist.

17. Vektor nach einem der Ansprüche 1 bis 11 als Arzneimittel nach Anspruch 16, bei welchem das Oligonukleotid in ein "Antisinn"-RNA-Molekül oder ein Ribozym-RNA-Molekül transkribiert wird, welche die Expression eines an einer Pathologie beteiligten Gens blockieren, indem sie mit dessen mRNA von zellulärem, viralem, bakteriellem oder parasitärem Ursprung hybridisieren und/oder diese schneiden.

18. Vektor, gebildet aus einem defekten viralen Vektor, wie einem Adenovirus, oder einem defekten retroviralen Vektor, wie einem Mäuse-Retrovirus, als Arzneimittel nach Anspruch 16 oder 17.

19. Vektor nach einem der Ansprüche 1 bis 11, welcher als Oligonukleotide insbesondere anti-rev- oder anti-tat-Antisinn-Sequenzen umfasst, als Arzneimittel für die Behandlung von AIDS nach einem der Ansprüche 16 bis 18.

**Claims**

1. Recombinant DNA vector containing a cassette for transcription by RNA polymerase III, consisting of the adenovirus VA I gene transcribed by RNA polymerase III, into which an oligonucleotide, a DNA fragment, has been inserted into the region ranging from nucleotides 10694 to 10730 of said VA1 gene of adenovirus 2 represented in Figure 1, or in place of the region ranging from nucleotides 10694 to 10730, said region being deleted.

2. Vector according to Claim 1, **characterized in that** said oligonucleotide is inserted into the region ranging from nucleotides 10702 to 10728 of said adenovirus 2 VA1 gene represented in Figure 1, or in place of said region ranging from nucleotides 10702 to 10728, said region being deleted, and the gene being referred to as VA delta IV.

3. Vector according to Claim 1 or 2, **characterized in that** the oligonucleotide is inserted at nucleotide 10711 of the adenovirus 2 VA-1 gene represented in Figure 1.

4. Vector according to one of the preceding claims, **characterized in that** the vector is a replicative plasmid or episomal vector or viral vector.

5. Vector according to one of the preceding claims, **characterized in that** the vector is an episomal vector carrying the origin of replication of the Epstein-Barr virus (oriP), and also the coding sequences for the EBNA 1 protein.

6. Vector according to one of the preceding claims, **characterized in that** it contains several identical or different virus genes transcribed by RNA polymerase III, into which an identical or different oligonucleotide has been inserted outside the boxes A and B in each of said viral genes.

7. Vector according to one of the preceding claims, **characterized in that** the oligonucleotide contains from 15 to 40 nucleotides, preferably 15 to 25.

8. Vector according to one of the preceding claims, **characterized in that** the adenoviral gene is a gene which has been inactivated, by deletion or mutation, as regards inhibition of interferon action.

9. Vector according to one of the preceding claims, **characterized in that** the oligonucleotide is inserted within the region responsible for the activity of inhibition of interferon action.

10. Vector according to one of the preceding claims, **characterized in that** it is a defective recombinant adenovirus.

11. RNA retroviral vector, **characterized in that** it contains the RNA transcript of the transcription cassette according to one of Claims 1 to 9.

12. Cells infected or transfected with a vector according to one of Claims 1 to 11.

13. Method for the intracellular production of an RNA fragment in vitro, in which eukaryotic cells containing RNA polymerase III are transfected or infected with a vector according to one of Claims 1 to 11, containing as oligonucleotides a DNA fragment corresponding to the reverse transcript of said RNA, and the eukaryotic cells thus transfected or infected are cultured in a suitable culture medium.

14. Method for blocking the expression of a gene ex vivo using a vector according to one of Claims 1 to 11, in which said oligonucleotide is transcribed to an antisense or ribozymal RNA molecule which hybridizes with and/or cleaves the messenger RNA of said gene.

15. Method for the treatment of cells ex vivo using a vector according to one of Claims 1 to 11.

16. As a medicinal product, a vector according to one of Claims 1 to 11, which can be used, in particular, as an antiviral, antitumour, antibiotic or antiparasitic agent, or in any pathology in which a gene is abnormally expressed, either by mutation or through deregulation.

17. As a medicinal product according to Claim 16, a vector according to one of Claims 1 to 11, in which the oligonucleotide is transcribed to an "antisense" RNA molecule or a ribozymal RNA molecule which blocks the expression of a gene involved in a pathology, by hybridizing with and, where appropriate, cleaving its messenger RNA of cellular, viral, bacterial or parasitic origin.

18. As a medicinal product according to Claim 16 or 17, a vector consisting of a defective viral vector such as an adenovirus or a defective retroviral vector such as a murine retrovirus.

19. As a medicinal product for the treatment of AIDS, according to one of Claims 16 to 18, a vector according to one of Claims 1 to 11, containing antisense sequences, in particular anti-rev or anti-tat sequences, as oligonucleotides.

# Figure 1: ADENOVIRUS 2   Gène VAI

```
      10569         10579         10589         10599         10609
CGTGCGCAGT CGTTGACGCT CTAGACCGTG CAAAAGGAGA GCCTGTAAGC

      10619         10629         10639         10649         10659
GGGCACTCTT CCGTGGTCTG GTGGATAAAT TCGCAACGGT ATCATGGCGG
                     BOX A

       10669         10679         10689         10699         10709
ACGACCGGGG TTCGAACCCC GGATCCGGCC GTCCGCCGTG ATCCATGCGG
                BOX B

       10719         10729         10739         10749         10759
TTACCGCCCG CGTGTCGAAC CCAGGTGTGC GACGTCAGAC AACGGGGGAG

      10769         10779         10789         10799         10809
CGCTCCTTTT GGCTTCCTTC CAGGCGCGGC GGCTGCTGCG CTAGCTTTTT

      10819
TGGCCACTGG
```

Figure 2 : séquences antisens et "random" insérées au nucléotide10711 du gène VAI.

.

anti-Tat   TGC TCT CCT CTG TCG AGT AAA GAC AGG ATA

anti-Rev   TCG TCG CTG TCT CCG CTT CTT CCT GCC A

Random    TGC TCT TGT CCC GTC ATC GTT GCC CCT C

5'                                                                                                    3'

ATC CAT GCG GTT ACC GCC CGC GTG

Gène VAI              10711

## FIG.3

**Analyse sur gel d'acrylamide/urée des produits de transcription acellulaire des constructions PVV2/VAI/antisens**

Tailles attendues:

VAI-ARN:  160 bp
VAI-ARN anti-rev:  188 bp
VAI-ARN anti-tat:  190 bp

FIGURE 4

Y-axis: % de cellules infectées par HIV (0, 20, 40, 60, 80, 100)

X-axis: Jours après infection HIV (0, 2, 4, 6, 8, 10, 12, 14, 16, 18, 20, 22)

Legend:
- AS1 (anti-tat)
- AS2 (anti-tat)
- AS3 (anti-rev)
- AS4 (anti-rev)
- CEM
- CEM néo (gén.r)

FIGURE 5
========

"anti-tat": AS1, AS2, AS6, AS7, AS8.
"anti-rev": AS3, AS4, AS9, AS10, AS12.
 CEM     :  témoin  positif d'infection

VA RNA₁

VA₁-crev RNA

FIG.6

FIGURE 7: PCR "overlap"

*site d'insertion*

Deux premières "demi-amplifications" séparées :
a et a', b et b':

Mélange des deux premières demi-amplifications
et nouvelle amplification avec a et b:

*séquence exogène insérée*

←—28 **kb** (régions tardives de l'Ad2)

←— 0.2 **kb** (ARN VA)

a: ARN de cellules AS10 exprimant la construction VA/anti-rev

b: ARN de cellules HepG2 infectées par de l' Adenovirus 2

# FIG.8

Northern-blot des ARN transcrits à partir d'un plasmide portant les gènes VA-anti rev et VA-anti tat

FIGURE 9

## A- Vecteur I:

- *dérivé du pMV7* :

## B- Vecteur II:

- *pMV7 dont les régions enhancers ont été délétées et remplacées par le ou les gènes VA-antisens.*
- *le gène de sélection peut être présent ou pas.*

Légendes:

→ ARN transcrits à partir des différents promoteurs .
▓ LTR
☐ gène de sélection
|VA/as| gène VA antisens

# FIG.10

FIG. 11